# EUROPEAN PATENT SPECIFICATION

(11) **EP 2 833 882 B1**
(45) Date of publication and mention of the grant of the patent: **24.07.2019**
(21) Application number: 13718524.5
(22) Date of filing: 05.04.2013
(51) Int. Cl.: A61K 31/215, A61K 31/22, A61K 31/225, A61P 21/06

(54) **KETONE BODIES AND KETONE BODY ESTERS FOR MAINTAINING OR IMPROVING MUSCLE POWER OUTPUT**
KETONKÖRPER UND KETONKÖRPERESTER ZUR BEWAHRUNG ODER VERBESSERUNG DER MUSKELLEISTUNG
CORPS CÉTONIQUES OU ESTERS DE CORPS CÉTONIQUES PERMETTANT DE MAINTENIR OU D'AMÉLIORER LA PRODUCTION DE PUISSANCE MUSCULAIRE

(30) Priority: 05.04.2012 GB 201206192
(43) Date of publication of application: 11.02.2015
(73) Proprietor: Tdeltas Limited, Thame, Oxfordshire OX9 3EZ (GB)
(72) Inventor: CLARKE, Kieran, Oxford Oxfordshire OX1 3PT (GB); COX, Peter, Oxford Oxfordshire OX1 3PT (GB)
(74) Representative: Geary, Stephen
(86) International application number: PCT/EP2013/057250
(87) International publication number: WO 2013/150153

(56) References cited:
- WO-A1-2010/021766
- WO-A1-2011/101171
- WO-A2-2004/108740
- WO-A2-2006/020137
- US-A- 5 693 850
- DATABASE MEDLINE [Online] US NATIONAL LIBRARY OF MEDICINE (NLM), BETHESDA, MD, US; March 1984 (1984-03), BOIARINOV G A ET AL: "[Effect of sodium oxybutyrate on macroergic phosphates, function and ultrastructure of the myocardium in blood loss].", XP002697484, Database accession no. NLM6704523 & BOIARINOV G A ET AL: "[Effect of sodium oxybutyrate on macroergic phosphates, function and ultrastructure of the myocardium in blood loss].", BIULLETEN' EKSPERIMENTAL'NOI BIOLOGII I MEDITSINY MAR 1984, vol. 97, no. 3, March 1984 (1984-03), pages 309-312, ISSN: 0365-9615
- DATABASE EMBASE [Online] ELSEVIER SCIENCE PUBLISHERS, AMSTERDAM, NL; 1981, OSTROVSKAYA R U ET AL: "Effect of prolonged administration of sodium hydroxybutyrate on the working capacity and muscle tissue in rats", XP002697485, Database accession no. EMB-1982034005 & OSTROVSKAYA R U ET AL: "Effect of prolonged administration of sodium hydroxybutyrate on the working capacity and muscle tissue in rats", FARMAKOLOGIYA I TOKSIKOLOGIYA 1981 SU, vol. 44, no. 5, 1981, pages 534-539, ISSN: 0014-8318
- DATABASE EMBASE [Online] ELSEVIER SCIENCE PUBLISHERS, AMSTERDAM, NL; May 2005 (2005-05), CLARK B M ET AL: "Dilated cardiomyopathy and acute liver injury associated with combined use of ephedra, [gamma]-hydroxybutyrate, and anabolic steroids", XP002697486, Database accession no. EMB-2005202899 & CLARK B M ET AL: "Dilated cardiomyopathy and acute liver injury associated with combined use of ephedra, [gamma]-hydroxybutyrate, and anabolic steroids", PHARMACOTHERAPY 200505 US, vol. 25, no. 5 I, May 2005 (2005-05), pages 756-761, ISSN: 0277-0008

## Description

This invention relates to the use of a ketone body ester for maintaining or improving muscle power output and to compositions for maintaining or improving muscle output. In particular, the invention relates to hydroxybutyrate esters for maintaining or improving muscle power output and which raise circulating ketone body concentrations in the blood plasma of a subject and especially to (R)-3-hydroxybutyrate-R-1,3-butanediol monoester.

Ketone bodies are produced when fatty acids levels are raised in the body and are metabolised by the body for energy. Ketone bodies have been disclosed as being suitable for reducing the levels of free fatty acids circulating in the plasma of a subject and that ingestion of ketone bodies can lead to various clinical benefits, including an enhancement of cognitive performance and treatment of cardiovascular conditions, diabetes and treatment of mitochondrial dysfunction disorders and in treating muscle fatigue and impairment.

A paper by Boiarinov, describes certain butyrates in *"*Effect of sodium oxybutyrate on macroergic phosphates function and ultrastructure of the myocardium in blood loss"; US National Library of Medicine, March 1984 (1984-03). The paper by Ostrovskaya *"Effect of prolonged administration of sodium hydroxybutyrate on the working capacity and muscle tissue in rats",* Embase Database Accession NoEMB-1982034005 also describes the function of a particular a hydroxybutyrate. The paper by Clark *"Dilated cardiomyopathy and acute liver injury associated with combined use of ephedra gamma-hydroxybutyrate and anabolic steroids.";* Embase Database Accession No EMB-2005202899, describes use of gamma hydroxybutyrate.

WO2004/108740 discloses compounds and compositions containing (R)-3-hydroxybutyrate derivatives effective for elevating blood concentrations of ketone bodies and methods for using such compounds particularly oligomers and compositions as nutritional supplements or for treating medical conditions. (R)-3-hydroxybutyrate derivatives and compositions that include these derivatives may serve as precursors to ketone bodies, such as acetoacetate and (R)-3-hydroxybutyrate, and are said to yield elevated blood concentrations of ketone bodies when administered to a subject.

WO2004/105742 discloses the use of a compound for example ketone bodies, salicylic acid, nicotinic acid, thiazolidine diones and fibrates, that reduces free fatty acids circulating in the blood plasma of a subject for the treatment or prevention of muscle, particularly cardiac or skeletal muscle impairment or fatigue or mitochondrial dysfunction. Liquid compositions for rehydration during or after exercise, comprising water, a sugar carbohydrate and a compound that reduces free fatty acids circulating in blood plasma are also disclosed.

WO2010/021766 discloses 3-hydroxybutyl 3-hydroxybutyrate enantiomerically enriched with respect to (3*R*)-hydroxybutyl (3*R*)-hydroxybutyrate ester which is an effective and palatable precursor to the ketone body (3*R*)-hydroxybutyrate.

Certain therapeutic and other benefits of ketone bodies are known and ketones may reduce free fatty acid levels in a patient or subject. We have now surprisingly found that ketone bodies and ketone body esters may maintain or improve muscle power output.

In a first aspect, the invention provides a ketone body ester comprising a monoester of (R)-3-hydroxybutyrate with an alcohol comprising R-1,3-butanediol in maintaining or improving the muscle power output of a healthy subject.

As used herein, the term "ketone", "ketone body" or "ketone bodies" means a compound or species which is a ketone or a ketone body precursor, that is, a compound or species which is a precursor to a ketone and which may be converted or metabolised to a ketone.

The invention maintains or improves skeletal muscle power output. It may also provide maintain or improve power output of other muscle types, for example cardiac muscle. Power output of muscles may be measured in accordance with the methods set out in the examples herein.

The invention is especially useful in maintaining or improving the skeletal muscle power output of a healthy person for example a person whose blood when tested does not show medical causes which may influence physical performance for example iron deficiency, haemoglobin (Hb), electrolytes, white cell count (WCC) and fasting glucose. The invention is particularly useful in maintaining or improving muscle performance in physically fit subjects, particularly in subjects having high levels of fitness for example athletes and military personnel, particularly elite athletes where improvements from an already high level of power output may still be improved.

The invention suitably provides an improved power output of at least 0.25%, preferably at least 0.5% and more preferably at least 1% relative to a placebo when measured in a controlled test as set out in the examples herein.

The invention in a preferred embodiment provides an increased power output of at least 1 Watt, more preferably at least 2 Watts and desirably at least 5 Watts relative to a placebo when measured in a controlled test as set out in the examples herein. The increased power output is suitably achieved after 30 minutes, more preferably after 15 minutes in the controlled test.

Any ketone body ester comprising a monoester of (R)-3-hydroxybutyrate with an alcohol comprising R-1,3-butanediol may be employed in the invention. In addition, other ketones or ketone esters or any compound which provides a ketone in the human body may also be employed. Examples of additional suitable ketone bodies or compounds which provide a ketone body *in situ* include hydroxybutyrates and derivatives thereof, for example esters of hydroxybutyrate including (R)-3-hydroxybutyrate and derivatives thereof, esters of (R)-3-hydroxybutyrate and oligomers of (R)-3-hydroxybutyrate including esters derived from alcohols and compounds containing one or more free hydroxyl groups. Suitable alcohols include butanediol, especially, butane-1,3- diol, altrose, arabinose, dextrose, erythrose, fructose, galactose, glucose, glycerol, gulose, idose, lactose, lyxose, mannose, ribitol, ribose, ribulose, sucrose, talose, threose, xylitol, xylose.

Whilst parenteral administration of ketone bodies is known, for example from US-A-6,136,862, oral administration is desirable in order to achieve a raised circulating ketone bodies level rapidly which with parental administration or injecting would not be feasible due to the volumes of a salts or acid that might be required. Other benefits of oral administration include convenience in not requiring parenteral administration or injection equipment, compliance of the subject with a dosing regimens and possible aversion of the subject to needles and for ease of administration, particularly where multiple doses of the ketone are to be ingested over relatively short intervals and where doses are consumed to provide improved athletic performance,.

Once ingested, the ketone body then needs to pass from the gut to the blood in order then to provide a physiological effect. The concentration of ketone in the blood depends on the level of uptake of the ketone from the gut to the blood. For ketones with a relatively low uptake, a higher level of ketone will be required in the gut. This in turn requires a greater volume of ketone to be ingested to achieve a given blood concentration of ketone.

Advantageously, we have found that (R)-3-hydroxybutyrate-R-1,3-butanediol monoester is surprisingly non-aversive to taste. Furthermore, this monoester provides a surprisingly high level of uptake thereby enabling high blood concentrations of hydroxybutyrate to be achieved upon consumption of an oral dose.

The invention is as defined in the appended claims.

Accordingly, the invention provides (R)-3-hydroxybutyrate-R-1,3-butanediol monoester for use in maintaining or improving the muscle power output of a subject.

In an especially preferred embodiment, the ketone comprises 3-hydroxybutyl-(R)-3-hydroxybutyrate, particularly in enantiomerically enriched form.

The invention also provides a ketone body ester comprising a monoester of (R)-3-hydroxybutyrate with an alcohol comprising R-1,3-butanediol, for use in raising blood (R)-3-hydroxybutyrate concentration to at least 1mM, preferably to at least 2mM, especially 3mM after oral administration to a subject of monoester at 0.5g/kg of body weight of the subject. Upon oral administration of a dose of monoester of 1g/kg of body weight of the subject, the blood (R)-3-hydroxybutyrate concentration is suitably at least 4mM, preferably at least 5mM, especially 6mM. Upon oral administration of a dose of monoester of 1.5g/kg of body weight of the subject, the blood (R)-3-hydroxybutyrate concentration is suitably at least 7mM, preferably at least 8mM, especially at least 9mM. The oral administration may be carried out in multiple doses but is preferably carried out in a single dose.

3-hydroxybutyl-(R)-3-hydroxybutyrate is particularly advantageous as it allows a large rise in blood hydroxybutyrate to be achieved with oral ingestion of a much smaller volume of material than with other ketones and other forms of delivery for example parenteral. A subject ingesting the material prior to or during physical exercise is accordingly much more readily able to ingest adequate ketone in order to provide a physiologically beneficial response without risk of physical discomfort either due to a large volume or bitter or otherwise aversive flavour. The high level of blood (R)-3-hydroxybutyrate concentration also maintains raised concentrations for a longer period than other ketones whereby to maintain raised levels, a lower frequency for administration of further doses is required than for other ketones.

Glycerol monoesters and diesters are also non-aversive to taste and provide raised blood (R)-3-hydroxybutyrate and (R)-3-hydroxybutyrate glycerol monoester or diester may be used in maintaining or improving the muscle power output of a subject. The monoester is suitably esterified at the 1 position. The diester is suitably esterified at the 1 and 3 positions. The (R)-3-hydroxybutyrate is monomeric.

(R)-3-hydroxybutyrate-R-1,3-butanediol monoester, (R)-3-hydroxybutyrate glycerol monoester or diester may be in combination with each other or include other ketone bodies or ketone body precursors in an amount less than any other such ketone bodies or preferably in an amount more than any such other ketone body. In an especially preferred embodiment (R)-3-hydroxybutyrate-R-1,3-butanediol monoester is the only ketone body or ketone body precursor present in the composition of the invention.

The invention provides in a further aspect, use of a composition comprising a monoester of (R)-3-hydroxybutyrate with an alcohol comprising R-1,3-butanediol in maintaining or improving the muscle power output of a healthy subject
Suitably the composition comprises water and a ketone body ester. Preferably, the composition comprises a ketone body precursor, a flavouring and optionally one or more of a protein, carbohydrate, sugars, fat, fibre, vitamins and minerals.

Different ketone bodies or ketone body esters have different levels of uptake. We have found that ketone esters are digested more effectively than other forms of ketone for example triolides and oligomers. In order to benefit from the relative ease of digestion, the ketone body preferably comprises a ketone ester. As a practical benefit, to achieve a given level of plasma ketone, the composition may contain a lower level of ketone ester than if another ketone body were to be used, so allowing ready ingestion and comfort prior to or during exercise such that improved muscle power delivery may be secured without discomfort or unpleasantness for the subject in ingesting a material whilst exercising. This is especially beneficial where the level of exercise is vigorous or prolonged. Advantageously, this allows a subject to ingest doses of the ketone body ester immediately before or during exercise to maintain or improve muscle power delivery.

Preferably, the ketone body ester comprises a partial ester, that is a polyol in which only a proportion of the hydroxyl groups are esterified. Monoesters are especially preferred and esters where two or more hydroxyl groups have been esterified but the esterified hydroxyl groups are not in a "beta-relationship, that is in which the hydroxyl groups are not attached to adjacent carbon atoms.

Hydroxybutyrate esters and especially partial esters for example hydroxybutyrate butane-1,3-diol monoester provide surprising levels of uptake and are accordingly especially suited for providing a subject with maintained or improved muscle power output.

Suitably the ketone body ester is ingested at a level of at least 100mg per kilogram of body weight of ketone per day. The blood plasma level of ketone will depend on the body mass of the individual and we have found that a ketone dose of at least 300 mg of ketone per kilogram of body weight provides a blood plasma concentration of ketone of around 1.5 mM. Desirably, the ketone body ester is ingested at a level adequate to provide a blood plasma ketone level of at least 0.1mM, preferably at least 0.2 mM, more preferably at least 1mM and optimally at least 2 mM. Suitably the ketone body ester is ingested at a level such that the blood plasma ketone level does not exceed 20mM, suitably does not exceed 10mM, or 8 mM and may not exceed 5mM.

Blood plasma levels of ketone may be determined by commercially available testing kits, for example, Ketostix, available from Bayer, Inc.

Accordingly in a preferred embodiment, the invention provides for use of (R)-3-hydroxybutyrate butane-1,3-diol monoester for use in maintaining or improving the muscle power output of a subject.

A further preferred embodiment provides a hydroxybutyrate ester or partial ester for example (R)-3-hydroxybutyrate butane-1,3-diol monoester for use in maintaining or improving the muscle power output of a subject wherein the circulating levels of hydroxybutyrate and acetoacetate in the blood of the subject are from 0.1 to 20, preferably 0.5 to 10 and optimally greater than 1 to 5mM.

The invention also provides for the use of a ketone body ester comprising a monoester of (R)-3-hydroxybutyrate with an alcohol comprising R-1,3-butanediol or a composition containing a ketone body ester comprising a monoester of (R)-3-hydroxybutyrate with an alcohol comprising R-1,3-butanediol in maintaining or improving the muscle power output of a subject in maintaining or improving the muscle power output of a healthy subject.

Preferably the circulating levels of hydroxybutyrate and acetoacetate in the blood of the subject are from 0.1 to 20, preferably 0.5 to 10 and optimally greater than 1 to 5mM.

Ketone bodies and ketone body esters are relatively unpalatable. We have found that esters of (R)-3-hydroxybutyrate, especially partial esters are less unpalatable than other ketone body ester and assist in providing adequate delivery of the desired ketone bodies to the subject at a sufficiently high level to provide the desired effects.

By selecting a certain combination of components including a ketone body containing a hydroxybutyrate ester and a flavouring, especially a bitter flavouring, an organoleptically acceptable composition which allows ketone to pass into the blood plasma at a desirable level may be obtained and which provide improved muscle power output.

By "organoleptically acceptable" we mean that the composition must possess acceptable sensory properties of taste, colour, feel and odour. The organoleptic acceptability or otherwise of the composition is a subjective assessment by the user having regard to external factors including personal taste and may be determined using blind tests.

Power output may be maintained or improved by administration of a a ketone body ester comprising a monoester of (R)-3-hydroxybutyrate with an alcohol comprising R-1,3-butanediol in a dose regime wherein the regime comprises administering in at least one dose of a ketone body ester to provide a circulating level of hydroxybutyrate and acetoacetate in the blood from 0.1 or 1 to 20, preferably 0.5 or 1 to 10 and optimally greater than 1 to 8mM or 1 to 5mM wherein the at least one dose comprises a ketone body ester in an amount of at least 100mg per kg of bodyweight of the subject per dose and preferably at least 300 to 750 mg/kg.

Where the a ketone body ester is provided as a composition in solid form, the level of a ketone body ester in the composition suitably comprises at least 5% by weight of ketone body including the hydroxybutyrate ester, more preferably at least 10% by weight and up to 95% by weight of the composition. Whilst a level of 15 to 30% by weight of the dry composition may be suitable, for example where the composition is a dry powder intended for use with a liquid to produce a liquid composition, a solid bar or product form suitably comprises from 30 to 95%, especially 50 to 95% by weight of the composition. Where the composition is in liquid form, the composition suitably comprises the ketone body at a level of at least 1%, for example 3 to 40% by weight of the liquid composition but may be higher for example up to 50% by weight of the composition depending on whether the composition is intended to be taken as a single dose or in multiple smaller doses to reach the desired blood ketone level.

The composition in liquid form suitably comprises the dry composition diluted with a suitable liquid, for example water, fruit juice or milk, preferably at a ratio of 1:1 to 1:10, more preferably 1:3 to 1:7 of dry composition to liquid. The level of ketone body which is organoleptically acceptable will vary according to the precise composition and its form and the masking effect of other components of the composition.

The composition may be solid, for example a powder, tablet, bar, confectionary product or a granule and intended for use as a solid oral dose form. In another embodiment, the solid composition may be mixed before use with a liquid, preferably water, fruit based liquid or a dairy product for example milk and yoghurt, to provide a liquid drink for the user. Milk, fruit juice and water are especially preferred as a carrier for the composition. The composition may be provided, as desired, as a liquid product in a form ready for consumption or as a concentrate or paste suitable for dilution on use. The diluent for use with the liquid composition is preferably milk, fruit juice or water.

When the composition is in solid form the composition may further comprise one or more of the following components:
- a diluent for example lactose, dextrose, saccharose, cellulose, corn starch or potato starch;
- a lubricant for example silica, talc, stearic acid, magnesium or calcium stearate and/or polyethylene glycols;
- a binding agent for example starches, arabic gums, gelatin, methylcellulose, carboxymethylcellulose, or polyvinyl pyrrolidone;
- a disintegrating agent such as starch, alginic acid, alginates or sodium starch glycolate;
- an effervescing agent;
- a dyestuff;
- a sweetener;
- a wetting agent for example lecithin, polysorbates, lauryl sulphates.

The composition may also be provided in encapsulated form provided that the encapsulation material and the quantity in which it is used is suitable for safe human consumption. However, encapsulation is not preferred.

A composition of the invention may contain a medium chain triglyceride (MCT) and optionally their associated fatty acids. MCTs comprise fatty acids with a chain length of between 5 and 12 carbon atoms. It is known that a diet rich in MCT results in high blood ketone levels. Suitable medium chain triglycerides are represented by the following formula CH₂R₁-CH₂R₂-CH₂R₃ wherein R1, R2 and R3 are fatty acids having 5 to12 carbon atoms. Preferably, MCTs wherein R1, R2, and R3 are fatty acids containing a six-carbon backbone (tri-C6:0) are employed as it is reported that tri-C6:0 MCT are absorbed very rapidly by the gastrointestinal track.

Where an MCT is employed, suitably the composition of the invention comprises i) a ketone body, preferably a ketone monoester, more preferably a (R)-3-hydroxybutyrate monoester and ii) a MCT, preferably tri-C6:0 MCT.

The composition of the invention may also comprise L-carnitine or a derivative of L-carnitine. Examples of derivatives of L-carnitine include decanoylcamitine, hexanoylcarnitine, caproylcarnitine, lauroylcarnitine, octanoylcarnitine, stearoylcarnitine, myristoylcarnitine, acetyl-L-carnitine, O-Acetyl-L-carnitine, and palmitoyl-L-carnitine. Where a carnitine is employed, suitably the composition of the invention comprises i) a ketone body, preferably a ketone monoester, more preferably a (R)-3-hydroxybutyrate monoester and ii) L-carnitine or a derivative of L-carnitine.

In a further embodiment, the composition may comprise i) a ketone monoester of (R)-3-hydroxybutyrate with an alcohol comprising R-1,3-butanediol, ii) a MCT, preferably tri-C6:0 MCT or a tri-C8:0 MCT and iii) L-carnitine or a derivative of L-carnitine.

Where MCT and L-carnitine or its derivative is employed, suitably the MCT is emulsified with the carnitine. Preferably 10 to 500 g of emulsified MCT is combined with 10 to 2000 mg of carnitine for example 50 g MCT (95% triC8:0) emulsified with 50 g of mono- and di-glycerides combined with 500 mg of L-carnitine.

The MCT may be present in a greater amount than the ketone body but preferably the level of ketone body is greater than the level of the MCT.

The composition may be in the form of a solid or in the form of a liquid composition or a gel. Suitable solid forms of the composition include a bar or powder suitable for mixing with a liquid, for example water, milk or fruit juice at the point of use. Suitable forms of liquid composition include for example a syrup, an emulsion and a suspension. Suitably, in the form of a syrup, the composition further may contain as carrier, for example, saccharose or saccharose with glycerol and/or mannitol and/or sorbitol. In the form of a suspension or emulsion, the composition may contain as a carrier, for example, a natural gum, agar, sodium alginate, pectin, methylcellulose, carboxymethylcellulose or polyvinyl alcohol.

The composition may also be a food product, food supplement, dietary supplement, functional food or a nutraceutical or a component thereof.

A food product is an edible material composed primarily of one or more of the macronutrients protein, carbohydrate and fat, which is used in the body of an organism to sustain growth, repair damage, aid vital processes or furnish energy. A food product may also contain one or more micronutrients such as vitamins or minerals, or additional dietary ingredients such as flavourants and colourants. The term food product as used herein also covers a beverage.

Examples of food products into which the composition may be incorporated as an additive include snack bars, cereals, confectionery and probiotic formulations including yoghurts. Examples of beverages include soft beverages, alcoholic beverages, energy beverages, dry drink mixes, nutritional beverages and herbal teas for infusion or herbal blends for decoction in water.

A nutraceutical is a food ingredient, food supplement or food product which is considered to provide a medical or health benefit, including the prevention and treatment of disease. In general a nutraceutical is specifically adapted to confer a particular health benefit on the consumer. A nutraceutical typically comprises a micronutrient such as a vitamin, mineral, herb or phytochemical at a higher level than would be found in a corresponding regular food product. That level is typically selected to optimise the intended health benefit of the nutraceutical when taken either as a single serving or as part of a diet regimen or course of nutritional therapy.

A functional food is a food that is marketed as providing a health benefit beyond that of supplying pure nutrition to the consumer. A functional food typically incorporates an ingredient such as a micronutrient as mentioned above, which confers a specific medical or physiological benefit other than a nutritional effect. A functional food typically carries a health claim on the packaging.

The invention provides in further aspect a kit comprising a product selected from a ketone body, a ketone body ester and a composition according to the invention and a ketone monitor and optionally instructions as to the level of product to consume per unit body weight to achieve a pre-determined level of blood plasma ketone and a dosage regimen to maintain blood plasma ketone at the pre-determined level to maintain or improve muscle power output. The user suitably consumes the product and may then periodically test their blood plasma ketone level to determine whether further ingestion of ketone is required to reach or to maintain the desired blood plasma ketone level.

Suitably the ketone body ester or composition comprising it is provided with instructions for consumption. Suitably the instructions to consume one or more doses of a ketone body ester or composition according to the invention per day or consume a dose prior to exercise, preferably at least 10 minutes, more preferably at least 30 minutes and optimally at least 1 hour prior to exercise.

For prolonged exercise, for example more than 20 minutes, 2 or more doses, more preferably 2 to 8 doses for example 3 or 6 doses may be consumed periodically to raise or to maintain raised blood plasma ketone levels. Suitably the doses are consumed at regular intervals as this maintains a more even level of blood ketone content although a user may consume a dose to maintain or improve power output so as to "prime" the blood with ketone.

The invention is described by reference to the following non-limiting examples.

### Example 1

### Methods:

### Subjects and recruitment

Athletes were recruited according to the qualification criteria defined by GB rowing for national trials. Both male and female athletes of all weight categories were included in the trial, with every effort made to recruit athletes of sub elite or elite calibre. All athletes were healthy, with no previous history of medical illness. Athletes had been training continuously for at least 12 weeks prior to testing, with all athletes regularly performing these tests within their training and competition schedules. Written informed consent was obtained from all athletes following an explanation of the risks associated with participation. All testing conformed to the standards of ethical practice as outlined in the declaration of Helsinki.

A baseline medical questionnaire, physical examination, body composition analysis and ECG were performed before any exercise testing. A baseline blood test was performed to exclude other possible medical causes which may influence performance, such as iron deficiency, Hb, electrolytes, WCC and fasting glucose.

**Table 1: Physical characteristics**

| **Athlete** | **HWM** | **LWM** | **HWW** | **LWW** |
|---|---|---|---|---|
| Age (yr) | 24.8 (+/-1) | 24.2 (+/-1.6) | 24.7 (+/-1.2) | 20.4 (+/-0.9) |
| Height (m) | 1.95 (+/-2.4) | 1.81 (+/-2.7) | 1.80 (+/-1.7) | 1.72 (+/-1.8) |
| Weight (kg) | 96.1 (+/-3) | 75.2 (+/-2.6) | 77.8 (+/-1.7) | 63.0 (+/-1.9) |
| Fat % | 12.5 (+/-1.1) | 10.1 (+/-1.5) | 23.7 (+/-1.9) | 16.1 (+/-2.9) |
| Lean mass (kg) | 83.8 (+/-1.9) | 67.8 (+/-3.4) | 56.8 (+/-4.2) | 52.8 (+/-1.3) |
| BMI (kg/m²) | 25.3 (+/-0.4) | 22.9 (+/-0.4) | 24.0 (+/-0.8) | 21.5 (+/-1.0) |
| Hb (g/L) | 148.2 (+/-0.3) | 152.3 (+/-0.2) | 137.5 (+/-0.5) | 127.0 (+/-0.2) |
| 2 km PB (min:s) | 6:04 (+/-3) | 6:24 (+/-2) | 7:05 (+/-6) | 7:20 (+/-4) |

| | | | | |
|---|---|---|---|---|
| *HWM:* Heavy weight Male; *LWMN*: Light weight Male; *HWW*: Heavy weight Women; *LWW*: Light weight Women | | | | |

### Experimental design and performance trial:

Subjects presented to the OCMR department, at the John Radcliffe hospital, Oxford following an overnight fast of at least 8 hours. Testing was performed at identical times of the day to reduce the effect of diurnal patterns on performance. Athletes were asked to consume an identical pre-testing meal the night before, as they would before a major competition, and to repeat this before every visit. Athletes were asked not to perform strenuous exercise in the 48 hours prior to each test, and to refrain from alcohol for 24 hours. Every effort was made to ensure that athletes were tested within the same macrocycle of training, and to standardise the timing of testing within a training week.

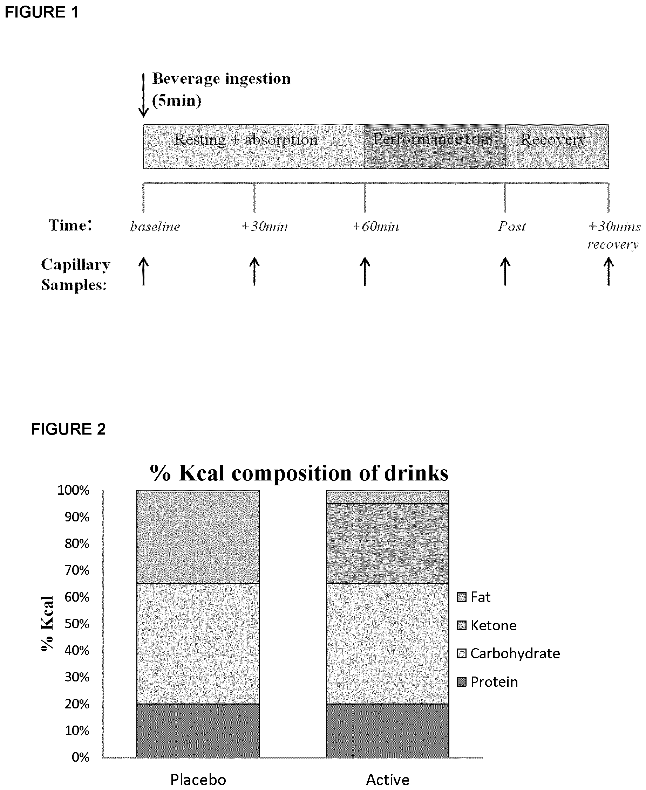

Figure 1 shows a schematic of protocol for each day of testing, showing time points for beverage ingestion, blood collection and performance trial. This protocol was repeated for placebo and active drinks.
On arrival subjects were weighed and their body composition recorded, before being escorted to a private room in the testing facility. Participants ingested body weight adjusted doses of beverage over 5 min and remained undisturbed for 60 min except for capillary blood samples at 30 min and 1 hour post ingestion.

All performance tests were conducted using a concept II rowing ergometer (Model D, Nottingham, UK) which participants all used as part of everyday training, and required no familiarisation of the testing apparatus. Testing was performed in a private area free of distractions, and open to free air flow. All tests were performed in ambient conditions between 17 and 22° C, with additional cooling provided by floor mounted fans to minimise thermal stress. Athletes were tested individually, in the presence of the same study investigator on each occasion. Verbal encouragement was provided if requested, otherwise testing was performed in silence. If music was requested by the athletes, an identical playlist was ensured on both trials. All capillary blood samples were obtained within 30 sec of exercise completion.

Performance data were automatically collected and stored in the Concept II ergometer (PM3 device) and offloaded in 5 min intervals during 30 min tests, or 500m during 2 km trials. Data was stored on a data chip and transferred to a study laptop for analysis. Real time data was displayed on a screen in front of the athlete to ensure adherence to stroke rates, with time or distance remaining, and 500m split time also displayed. Athletes were forbidden from using the pacing boat function.

### Drink preparation and dosing:

Each athlete received 2.083 g/kg body weight of raw drink powder mixed at a ratio of 1:1.5 parts water in a food blender for 2 min. The active ketone drink contained a (R)-3-hydroxybutyrate (R)-1,3-butanediol monoester contained the raw ketone ester at a dose calculated to achieve a peak ketonaemia of 3 mM of β-hydroxybutyrate, equivalent to several days of total fasting. Placebo drinks were identical in taste, viscosity and colour to ketone, with the calorific equivalent of ketone replaced with long chain triglyceride in the placebo preparations. As a powder the (% dry weight) compositions of milkshakes were as follows: Carbohydrate 45%, Ketone 30%, Protein 20%, and Fat (5% for active drink or 35% for placebo). All subjects were blinded to drink allocation, and consumed drinks in a 5 min time interval, after which subjects rested for 60 min to allow digestion and minimise gastric distress.

Figure 2 shows the percentage composition of study beverages. Active drink contained the ketone monoester, with identical composition of carbohydrate and protein in the placebo. Calories derived from the ketone monoester were replaced with fat in the placebo drink.

### Blood sampling:¹

Capillary blood samples (10-50µL) were obtained via an arterialised finger prick (Accuchek™), and immediately analysed for glucose, ketone and lactate using handheld monitors (Optium Xceed®, Abbott, UK and lactate Pro, Arkray, Japan).

### Resting ketone kinetics:

To evaluate the effects of exercise on the profile of blood ketone concentration, n = 7 representative age and sex matched athletic controls (n = 6 male and n = 1 female) were selected to undertake identical administration and absorption regimes of ketone containing beverage during resting sedentary conditions to enable comparison of resting vs. exercise changes in blood ketone concentration.

### Statistics:

Performance outcomes (distance in m, and seconds to complete 2 km trial) were analysed using paired student t-tests (two tailed). Comparisons of exercise vs. control blood ketone kinetics were made with a two way repeated measures ANOVA, with significant effects analysed using Bonferroni Post hoc correction.

Data evaluation was performed using SPSS (Version 17, Chicago, IL) with statistical significance set at the p < 0.05 level. All data is presented as means +/- standard error of the mean, and represents the pooled averages of n = 22 for 30 min trial and n = 13 for 2 km time trial.

Two subjects in total were excluded from all analysis. One participant was excluded on the basis of intercurrent illness, and another due to vomiting during the 30 min trial with subsequent mechanical interference with testing apparatus. Neither result, if included would alter any positive or negative findings of the study.

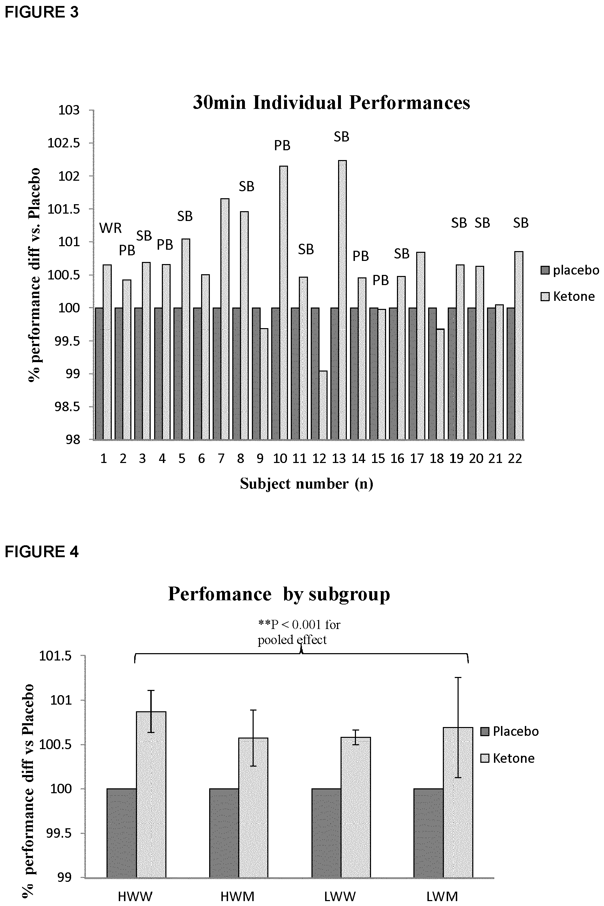

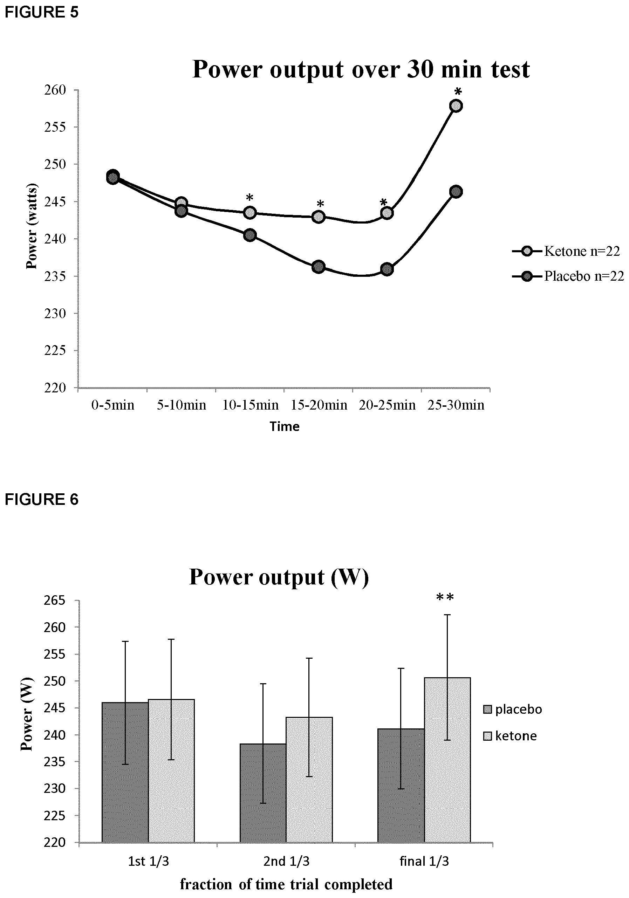

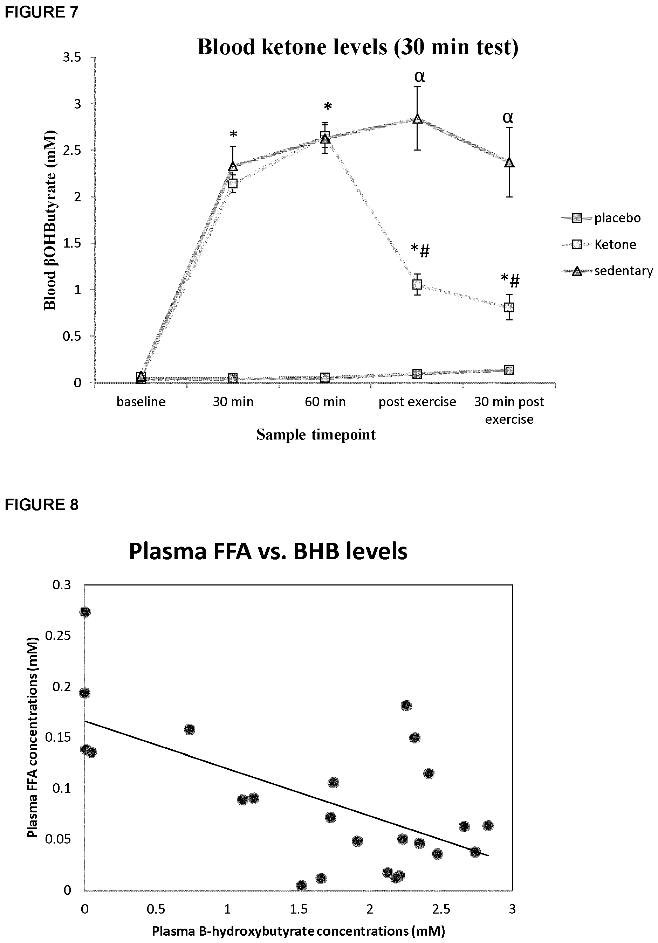

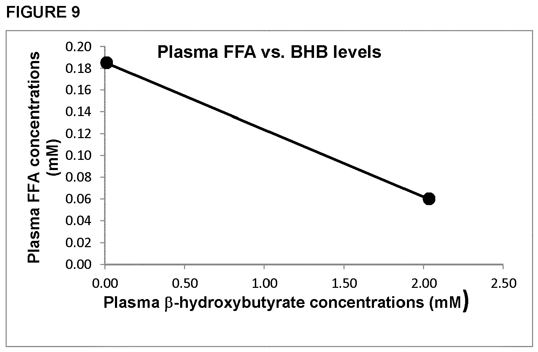

The results of these tests are shown in Figures 3 to 9.
Figure 3 shows individual performance profiles relative to placebo (expressed as a %). WR World record performance, PB Personal best performance, SB Seasons best performance.
Figure 4 shows performance effect for 30 min trial by subgroup of weight category relative to placebo (expressed as %). ** Pooled effect p < 0.001. Data expressed as means +/- SE.
Figure 5 shows power output over a 30 minute test and the beneficial effects of the ketone in providing improved skeletal power output as compared to placebos.
Figure 6 shows power outputs during 30 min test pooled for thirds of the time trial completed. A significant difference in the ketone arm was observed relative to placebo drink after the first 10 min of the trial. Data expressed as means +/- SE.
Figure 7 shows changes in blood levels of ketone over the duration of each exercise trial. Ingestion of ketone resulted in marked elevation of blood β-OH Butyrate concentrations compared to placebo (* p < 0.0001 for all time points). Ketone concentrations fell markedly by the end of the exercise trial compared to pre exercise values (# p< 0.0001). During exercise ketone concentrations fell significantly compared with resting control subjects not performing exercise (α p < 0.001).
Figure 8 shows Changes in plasma free fatty acid concentrations before and every 15 minutes for 90 minutes following ingestion of 0.5 g/kg body weight of ketone (n = 4 subjects).
Figure 9 shows Plasma free fatty acid concentrations before and 60 minutes following ingestion of 0.5 g/kg body weight of ketone (4 subjects).

### Example 2

Procedures were carried out to determine the uptake of oligomeric ketones into the blood. A blood level of (R)-3-hydroxybutyrate of between 0.1 to 0.2 mM was observed and was unlikely to produce a physiologically relevant response. R 1,3 butandiol - triolide diester produced a peak levels of about 1.5 mM. R 1,3 butandiol (R)-3-hydroxybutyrate monoester produced peak blood levels of 6 mM.

The following conclusions could be drawn from these tests:
1) Water soluble linear oligomers in the 6-7 mer range produce only minimal elevation of blood bHB.
2) Solid water insoluble linear oligomers in the 8-9 mer range raised blood bHB to 0.18 mM extending over 6 hours.
3) None of the linear oligomers appear to produce physiologically relevant elevation of blood ketones and the short chain oligomers are associated with gastric toxicity
4) The R 1,3 butandiol di triolide ester, is functionally 7 mer units long, produces blood levels is aversive to taste.

### Example 3

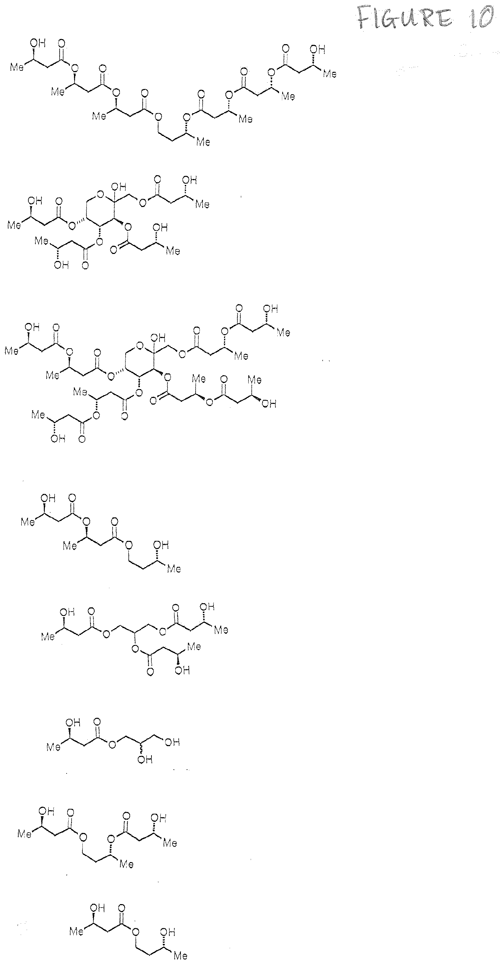

The flavour of various ketone bodies was tested. The ketone bodies, in volumes of -100 microlitres, were tasted by a tasting panel of 8 tasters. The taste of the ketones was assessed on a qualitative basis to assess their bitterness. The following Table 2 shows the ketone bodies tested and the structures of these ketones are shown in Figure 10.

**Table 2 - Ketones tested**

| **Ketone** | **Type** | **Number of hydroxyls** | **(R)-3-hydroxy butyrate residues** | **Ester bonds** |
|---|---|---|---|---|
| 1 | Oligomer - 3mer/diester | (R)-1,3-butanediol / 2 | 3 | 2 |
| 2 | Monomer -tetraester | Galactose/5 | 1 | 4 |
| 3 | Oligomer - 2 mer/tetraester | Galactose/5 | 2 | 4 |
| 4 | Oligomer - 2 mer/monoester | (R)-1,3-butanediol / 2 | 2 | 1 |
| 5 | Monomer - triester | Glycerol/3 | 1 | 3 |
| 6 | Monomer - monoester | Glycerol/3 | 1 | 1 |
| 7 | Monomer - diester | (R)-1,3-butanediol / 2 | 1 | 2 |
| 8 | Monomer - monoester | (R)-1,3-butanediol / 2 | 1 | 1 |

The results of the tests are set out in Table 3:

**Table 3 - Results**

| **Ketone** | **Flavour** |
|---|---|
| 1 | Bitter/aversive |
| 2 | Bitter/aversive |
| 3 | Bitter/aversive |
| 4 | Bitter/aversive |
| 5 | Bitter/aversive |
| 6 | Not bitter |
| 7 | Not bitter but aversive/unpleasant |
| 8 | Not bitter |

The results show that:
- the oligomers (Ketones 1, 3 and 4) having respectively 2 and 3 (R)-3-hydroxybutyrate were all perceived to be bitter;
- certain monomers (Ketones 2 and 5) were considered to be bitter and another (Ketone 7) was considered to be very unpleasant although not bitter. The monomers are respectively a tetraester, trimester and a diester;
- esters of (R)-1,3-butanediol (Ketones 1 and 4) were considered to be bitter and another ester of (R)-1,3-butanediol (Ketone 7) was considered to be very unpleasant although not bitter and another ester (Ketone 11) had an acceptable flavour;
- a monoester of (R)-1,3-butanediol (Ketone 4) was perceived to be bitter;
- the only ketones having acceptable flavour were Ketones 6 and 8, a glycerol monoester and a monoester of (R)-1,3-butanediol, respectively.

Lack of bitterness of an ester was not predictable based on a consideration of the large number of variables (number of butyrate groups, type of alcohol, number of ester bonds, different esters of a specified alcohol) before carrying out these tests. Even with the results, the effect of these factors is mixed. The oligomers of esters tested were all bitter (although longer chains of hydroxybutyrate may be less bitter), some monoesters were bitter but others were not, some esters of (R)-1,3-butanediol were bitter and one was acceptable and esters of different alcohols had an acceptable flavour.

### Example 4

Experiments were carried out to determine the concentration of blood (R)-3-hydroxybutyrate in rats following administration of various ketones. The ketones tested are set out in Table 4 below. The ketones were tasted in the same manner as Ketones 1 to 8 above.

Rats were each fed with a sour cream mix containing 2g of a ketone as set out in Table 3 in 10ml water. Samples of blood from each rat were taken immediately prior to feeding, after 1 hour, 3 hours and 6 hours to determine the blood concentration of (R)-3-hydroxybutyrate. Two controls (Control A and Control B) were also tested by feeding with the same mix without the ketone added. The rats were humanely killed and dissected for visual inspection to determine whether pyloric constriction or other visible changes had occurred.

Blood was extracted and 0.1ml of whole blood was added to 0.4ml 3.6% perchloric acid subjected to vortex and then allowed to rest in ice for 30minutes and then spun. 350 ul of acidic supernatant were then transferred to a new tube and 5ul phenol red (0.05%), 20 ul 1 M Imidazole pH 7 and 3 M KHCO3 was added to neutralize the solution which was allowed to stand in ice for 30 minutes and then spun. A sample of the neutralized blood extract was then assayed for (R)-3-hydroxybutyrate. The ketones tested are set out in Table 4:

**Table 4 - Ketones**

| **Ketone** | **Type** | |
|---|---|---|
| 9 | 8-10 Oligomer (8 and 9 mer, small amount 10 mer, no 7 mer or lower) | Solid, water insoluble |
| 10 | 7 mer equivalent - (R)-1,3-butanediol ditriolide ester | Dark yellow oil |
| 11 | 5-7 Oligomer (6 mer and 7 mer (less), less 5 mer, low amount of smaller mers) | Slight yellow oil, water soluble |
| 12 | 3-8 Oligomer (6 and 7 mer (equal amounts), half amount 8 mer, significant amounts of 3, 4, 5 mers) | Yellow oil, water soluble |
| 13 | Monomer ((R)-3-hydroxybutyrate-(R)-1,3-butanediol) | Same as Ketone 8 |

The results of these experiments are set out in Table 5:

**Table 5 - Results**

| **Ketone** | **Peak Blood (R)-3-hydroxybuty rate (mM)** | **Symptoms** | **Flavour** |
|---|---|---|---|
| 9 | 0.2 | No pyloric constriction, gas, yellow turds | Tasteless |
| 10 | 1.5 | No pyloric constriction, no gas | Aversive/unpleasant |
| 11 | 0.2 | Gas | Acidic but non-aversive |
| 12 | 0.2 | Least pyloric constriction | Acidic but non-aversive |
| 8/13 | 6 | No pyloric constriction, no gas | Not bitter, non-aversive |
| Control A (no ketone) | 0.05-0.1 | No pyloric constriction, no gas | |
| Control B (no ketone) | 0.05-0.1 | No pyloric constriction, no gas | |

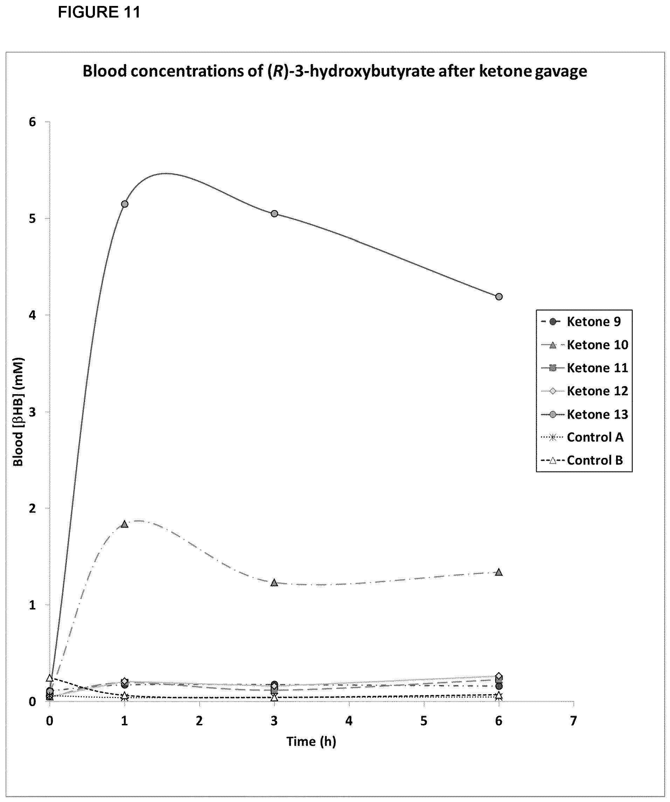

The concentration of (R)-3-hydroxybutyrate derived from administration of each ketone was plotted and is shown in Figure 11.

The results of these experiments show that:
- Oligomers in the 3-8, 5-7 and 8-10 mer range (Ketones 9, 11 and 12) provide blood levels of (R)-3-hydroxybutyrate of between 0.1 to 0.2 mM and is unlikely to produce a physiologically relevant response;
- Ketone 10 (R 1,3 butanediol triolide diester) produced peak levels of about 1.5 mM but had an aversive flavour;
- Ketone 13 of the invention (same as Ketone 8 above) provided peak blood levels of 6 mM;
- For Ketone 9 (8-10 mer), the rat turds produced were yellow which suggests the ketone traversed gut without being degraded or taken up into the blood to a material degree;
- The shorter chain oligomers are associated with significant acute gastric toxicity.

Uptake of the ketone was not predictable based on a consideration of the structures of the Ketones 9 to 13. The longer oligomers were tasteless and did not show symptoms of acute gastric toxicity but were not taken up in the blood to a physiologically relevant extent. Shorter oligomers were also not taken up to a material degree, were aversive to taste and caused acute gastric toxicity. The triolide diester of (R)-1,3-butanediol (Ketone 10) showed a level of uptake which may provide a physiological response, this level of uptake being around 10 times that of the other oligomers tested. However, this ketone was aversive to taste.

Ketone 18/13 ((R)-3-hydroxybutyrate-(R)-1,3-butanediol) shows a level of uptake to the blood which is between 30 and 40 times that of the linear oligomers tested (Ketones 9, 11 and 12) and around 4 times that of the triolide diester oligomer (Ketone 10). In 11, this vast difference in the level of uptake is shown graphically with the concentration of Ketone 13 rising to 6mM.

### Example 5

(R)-3-hydroxybutyrate-R-1,3-butanediol monoester was administered in a single dose based on a fixed amount per kg of body weight of the subject to a number of subjects. The blood (R)-3-hydroxybutyrate concentration was determined over a period of time to determine the dose/response relationship between the oral single dose and the blood concentration of (R)-3-hydroxybutyrate. The procedure was repeated with different quantities of (R)-3-hydroxybutyrate-R-1,3-butanediol monoester to provide data on the dose/response relationship for (R)-3-hydroxybutyrate-R-1,3-butanediol monoester when administered at 192mg/kg, 291mg/kg, 395mg/kg and 573mg/kg.

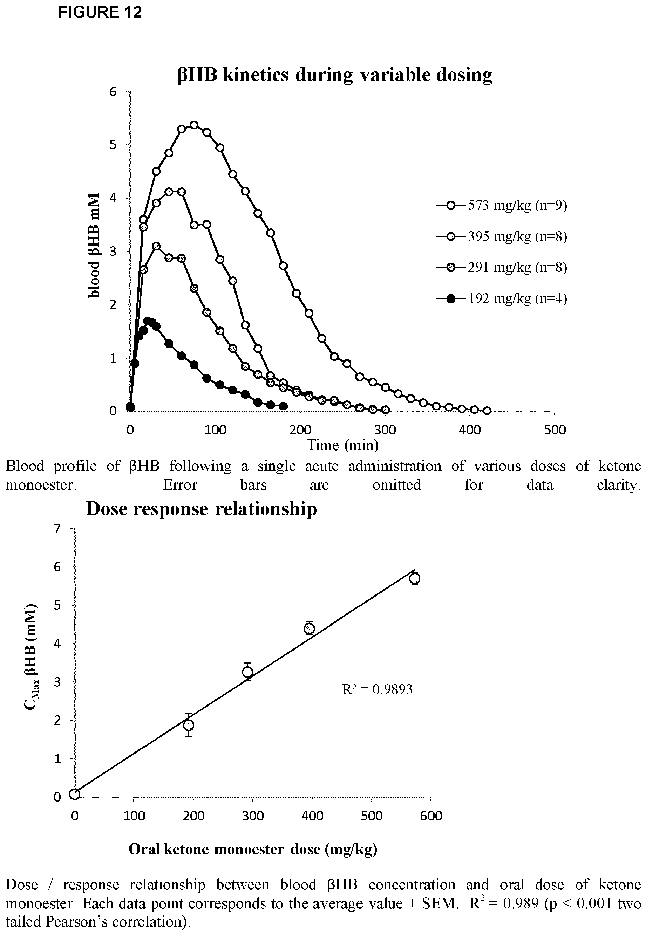

The results are shown graphically in Figure 12 and demonstrate that single dose of a small amount of (R)-3-hydroxybutyrate-R-1,3-butanediol monoester raises blood (R)-3-hydroxybutyrate concentrations to levels not previously reported with other ketones and which are orders of magnitude greater than generally achievable with (R)-3-hydroxybutyrate oligomers of R-1,3-butanediol.

## Claims

1. Use of a ketone body ester comprising a monoester of (R)-3-hydroxybutyrate with an alcohol comprising R-1,3-butanediol in maintaining or improving the muscle power output of a healthy subject.

2. Use according to claim 1 in maintaining or improving skeletal muscle power output.

3. Use according to any one of the preceding claims which provides an improved power output of at least 0.25% relative to a placebo when measured in a controlled test over 30 minutes.

4. Use according to any one of the preceding claims which provides an increased power output of at least 1 Watt relative to a placebo when measured in a controlled test over 30 minutes.

5. Use according to any one of the preceding claims wherein the circulating levels of hydroxybutyrate and acetoacetate in the blood of the subject are from greater than 1 to 5mM.

6. Use of a composition comprising a monoester of (R)-3-hydroxybutyrate with an alcohol comprising R-1,3-butanediol in maintaining or improving the muscle power output of a healthy subject.

7. Use of a composition according to claim 6 comprising water and optionally comprising one or more of a flavouring, a protein, carbohydrate, sugars, fat, fibre, vitamins and minerals.

8. Use of a composition according to claim 6 or 7 which further comprises a mid-chain triglyceride.

9. Use of a composition according to claim 8 wherein the composition comprises a mid-chain triglyceride having a formula CH₂R₁-CH₂R₂-CH₂R₃ wherein R1, R2 and R3 are fatty acids having 5 to12 carbon atoms.

## Patentansprüche

1. Verwendung eines Ketonkörperesters umfassend einen Monoester von (R)-3-Hydroxybuttersäure mit einem Alkohol, umfassend R-1,3-Butandiol, zur Bewahrung oder Verbesserung der Muskelleistung einer gesunden Person.

2. Verwendung gemäß Anspruch 1 zur Bewahrung oder Verbesserung der Skelettmuskelleistung.

3. Verwendung gemäß einem der vorhergehenden Ansprüche, die eine Leistungsverbesserung um wenigstens 0,25% bezogen auf ein Placebo bereitstellt, wenn in einem kontrollierten Test über 30 Minuten gemessen wird.

4. Verwendung gemäß einem der vorhergehenden Ansprüche, die eine Leistungserhöhung um wenigstens 1 Watt bezogen auf ein Placebo bereitstellt, wenn in einem kontrollierten Test über 30 Minuten gemessen wird.

5. Verwendung gemäß einem der vorhergehenden Ansprüche, wobei die zirkulierenden Gehalte an Hydroxybuttersäure und Acetoessigsäure im Blut der Person größer als 1 bis 5 mM sind.

6. Verwendung einer Zusammensetzung umfassend einen Monoester von (R)-3-Hydroxybuttersäure mit einem Alkohol, umfassend R-1,3-Butandiol, zur Bewahrung oder Verbesserung der Muskelleistung einer gesunden Person.

7. Verwendung einer Zusammensetzung gemäß Anspruch 6, umfassend Wasser und gegebenenfalls umfassend eines oder mehrere aus einem Geschmackstoff, einem Protein, Kohlenhydrat, Zuckern, Fett, Fasern, Vitaminen und Mineralien.

8. Verwendung einer Zusammensetzung gemäß Anspruch 6 oder 7, welche außerdem ein mittelkettiges Triglycerid umfasst.

9. Verwendung einer Zusammensetzung gemäß Anspruch 8, wobei die Zusammensetzung ein mittelkettiges Triglycerid mit Formel CH₂R₁-CH₂R₂-CH₂R₃ umfasst, wobei R₁, R₂ und R₃ Fettsäuren mit 5 bis 12 Kohlenstoffatomen sind.

## Revendications

1. Utilisation d'un ester de corps cétonique comprenant un monoester de (R)-3-hydroxybutyrate avec un alcool comprenant du R-1,3-butanediol dans le maintien ou l'amélioration de la production de puissance musculaire d'un sujet sain.

2. Utilisation selon la revendication 1 dans le maintien ou l'amélioration de la production de puissance musculaire squelettique.

3. Utilisation selon l'une quelconque des revendications précédentes qui fournit une production de puissance améliorée d'au moins 0,25 % par rapport à un placebo lorsqu'elle est mesurée dans un essai contrôlé en l'espace de 30 minutes.

4. Utilisation selon l'une quelconque des revendications précédentes qui fournit une production de puissance augmentée d'au moins 1 Watt par rapport à un placebo lorsqu'elle est mesurée dans un essai contrôlé en l'espace de 30 minutes.

5. Utilisation selon l'une quelconque des revendications précédentes dans laquelle les niveaux d'hydroxybutyrate et d'acétoacétate en circulation dans le sang du sujet sont de plus de 1 à 5 mM.

6. Utilisation d'une composition comprenant un monoester de (R)-3-hydroxybutyrate avec un alcool comprenant du R-1,3-butanediol dans le maintien ou l'amélioration de la production de puissance musculaire d'un sujet sain.

7. Utilisation d'une composition selon la revendication 6 comprenant de l'eau et facultativement comprenant un ou plusieurs d'un aromatisant, d'une protéine, d'un glucide, de sucres, de graisses, de fibres, de vitamines et de minéraux.

8. Utilisation d'une composition selon la revendication 6 ou 7 qui comprend en outre un triglycéride à chaîne intermédiaire.

9. Utilisation d'une composition selon la revendication 8 dans laquelle la composition comprend un triglycéride à chaîne intermédiaire ayant une formule CH₂R₁-CH₂R₂-CH₂R₃ dans laquelle R1, R2 et R3 sont des acides gras ayant 5 à 12 atomes de carbone.
